# EUROPÄISCHE PATENTSCHRIFT

(11) **EP 2 098 194 B1**
(45) Veröffentlichungstag und Bekanntmachung des Hinweises auf die Patenterteilung: **15.06.2011**
(21) Anmeldenummer: 09150368.0
(22) Anmeldetag: 12.01.2009
(51) Int. Cl.: A61F 2/84, A61F 2/00, A61F 2/02, A61M 25/10

(54) **Katheter und System zum Einbringen einer intraluminalen Endoprothese**
Catheter and system for delivering an intraluminal endoprosthesis
Cathéter et système d'introduction d'une endoprothèse intraluminale

(30) Priorität: 05.03.2008 DE 102008012744
(43) Veröffentlichungstag der Anmeldung: 09.09.2009
(73) Patentinhaber: Biotronik VI Patent AG, 6341 Baar (CH)
(72) Erfinder: Surber, Bettina, 8600, Dübendorf (CH); Nef, Thomas, 8173, Ried b. Neerach (CH)
(74) Vertreter: Lindner-Vogt, Karin L.

(56) Entgegenhaltungen:
- EP-A- 1 327 422
- US-A1- 2007 250 149
- US-B1- 6 702 802

## Beschreibung

Die vorliegende Erfindung bezieht sich auf einen Katheter mit einem Innenschlauch und einem den Innenschlauch zumindest abschnittsweise umgebenden Außenschlauch, wobei der Außenschlauch mit einem Ballon verbunden ist, der einen Endoprothesenabschnitt aufweist, welcher zur Anordnung einer intraluminalen Endoprothese dient. Die Erfindung bezieht sich ferner auf ein System zum Einbringen einer intraluminalen Endoprothese, vorzugsweise eines Stents, in einen Körperhohlraum bestehend aus der intraluminalen Endoprothese und einem derartigen Katheter.

Katheter sind Röhrchen oder Schläuche verschiedenen Durchmessers, die in den jeweiligen zu behandelnden Körperhohlraum eingeführt werden können. Sogenannte Ballonkatheter, die vor allem in der Angioplastie zur Erweiterung oder Wiedereröffnung eines Gefäßes eingesetzt werden, weisen einen Führungsdraht auf, der zuerst in das zu behandelnde Gefäß eingeschoben wird. Anschließend wird ein Schlauch, der in einem vorgegebenen Bereich des Schlauchs einen nicht dilatierten, gefalteten Ballon aufweist, entlang des Führungsdrahts bis zu der zu behandelnden Stelle des Gefäßes vorgeschoben, so dass der Ballon im Bereich der zu behandelnden Stelle des Gefäßes, die z. B. eine Stenose aufweist, platziert ist. Danach wird der Ballon dilatiert, d. h. entfaltet und aufgedehnt, so dass die zu behandelnde Stelle wiedereröffnet oder erweitert wird und der Strom der Körperflüssigkeit in dem Gefäß nicht mehr oder nicht mehr in dem zuvor vorliegenden Maße behindert wird. Schließlich wird der Ballon wieder entleert und entlang des Führungsdrahts wieder aus dem Gefäß entfernt. Gleichzeitig oder anschließend wird auch der Führungsdraht aus dem Gefäß zurückgezogen.

Heute verwendete Katheter weisen, um optimale Eigenschaften im Hinblick auf Biegsamkeit und Pushability zu erreichen, häufig einen Innen- und einen den Innenschlauch zumindest abschnittsweise umgebenden Außenschlauch auf. Als "Pushability" wird die Eigenschaft eines Katheters beschrieben, longitudinale Kräfte vom proximalen Ende des Katheters zu seinem distalen Ende, ohne Knicke auszubilden, zu übertragen. Innen- und Außenschlauch bestehen dabei aus gleichen oder unterschiedlichen Materialien, beispielsweise PA. Der Ballon des Katheters ist mit dem distalen Ende des Außenschlauchs des Katheters verbunden, insbesondere auf diesen aufgeschweißt.

Ballonkatheter werden häufig nicht nur zu Dilatation eines Gefäßes eingesetzt, sondern auch dazu verwendet, intraluminale Endoprothesen an eine zu behandelnde Stelle in einem Körperhohlraum einzubringen. Hierfür weist der Ballon eines derartigen Katheters einen Endoprothesenabschnitt auf, der zur Anordnung einer intraluminalen Endoprothese vorgesehen ist. Bei dem Endoprothesenabschnitt handelt es sich um den zylindrischen Abschnitt des Ballons, der nicht länger sein muss als die Endoprothese. Dieser Abschnitt ist ggf. etwas länger, jedoch dann nur geringfügig, als die Endoprothese. Die intraluminale Endoprothese, vorzugsweise ein Stent, wird in diesem Abschnitt auf den Ballon des Katheters aufgecrimpt und als System mit diesem gemeinsam in den Körperhohlraum eingeführt. Wenn die Endoprothese nach dem Einführen die vorgesehene Stelle erreicht hat, wird die Endoprothese zusammen mit dem Ballon des Katheters gedehnt und verbleibt nach dem Entleeren und Einfalten des Ballons in dem behandelten Gefäß.

Intraluminale Endoprothesen, vorzugsweise in Form von Stents, werden derzeit weit verbreitet eingesetzt, da sie eine einfache und kostengünstige Behandlung von Gefäßerkrankungen ermöglichen. Sie weisen häufig ein rohrförmiges oder hohlzylinderförmiges Grundgitter auf, das an beiden Längsenden offen ist. Das Grundgitter einer derartigen Endoprothese wird mittels eines Katheters in den zu behandelnden Körperhohlraum eingesetzt und dient nach Entfernen des Katheters dazu, den Körperhohlraum zu stützen. Durch den Einsatz von Stents können verengte Bereiche in den Gefäßen dauerhaft oder zumindest über einen bestimmten Zeitraum erweitert werden, so dass ein Lumengewinn im Körperhohlraum resultiert.

Intraluminale Endoprothesen werden heute häufig ebenfalls mit pharmazeutisch aktiven Substanzen versehen, die über einen bestimmten Zeitraum im Organismus freigesetzt werden.

Diese pharmazeutisch aktiven Substanzen können beispielsweise dazu dienen, Restenosen oder Agglomerationen zu vermeiden. Durch die Freisetzung von pharmazeutisch aktiven Substanzen, mit denen derartige intraluminale Endoprothesen versehen sind, ist es möglich, lediglich eine lokale Behandlung, d. h. eine Elution eines Wirkstoffs im Wesentlichen nur in das die intraluminale Endoprothese umgebende Gewebe, vorzunehmen. Dieser Vorgang wird auch als "Local Drug Delivery" (LDD) bezeichnet. Der Behandlungsort, an dem der Wirkstoff seine pharmakologische Wirkung entfalten soll, grenzt somit unmittelbar an den Ort der Implantation der intraluminalen Endoprothese.

Unter einer "pharmazeutisch aktiven Substanz" (oder therapeutisch aktive oder wirksame Substanz) im Sinne der Erfindung wird ein pflanzlicher, tierischer oder synthetischer Wirkstoff (Medikament) oder ein Hormon verstanden, das in geeigneter Dosierung als Therapeutika zur Beeinflussung von Zuständen oder Funktionen des Körpers, als Ersatz für natürlich vom menschlichen oder tierischen Körper erzeugte Wirkstoffe, wie Insulin, sowie zur Beseitigung oder zum Unschädlichmachen von Krankheitserregern, Tumoren, Krebszellen oder Körperfremdstoffen Einsatz findet. Die Freisetzung der Substanz in der Endoprothesenumgebung hat einen positiven Effekt auf den Heilungsverlauf oder wirkt pathologischen Veränderungen des Gewebes in Folge des chirurgischen Eingriffs entgegen bzw. dient dem Unschädlichmachen von maladen Zellen in der Onkologie.

Derartige pharmazeutisch aktive Substanzen weisen beispielsweise eine antiinflammatorische und/oder antiproliferative und/oder spasmolytische Wirkung auf, wodurch beispielsweise Restenosen, Entzündungen oder (Gefäß-)Spasmen vermieden werden können. Derartige Substanzen können in besonders bevorzugten Ausführungsbeispielen aus einer oder mehreren Substanzen der Wirkstoffgruppe der Calciumkanalblocker, der Lipidregulatoren (wie beispielsweise Fibrate), der Immunsuppressiva, der Calcineurininhibitoren (wie beispielsweise Tacrolimus), der Antiflogistika (wie beispielsweise Cortison oder Dichlofenac), der Antünflammatorica (wie beispielsweise Imidazole), der Antiallergika, der Oligonucleotide (wie beispielsweise dODN), der Östrogene (wie beispielsweise Genistein), der Endothelbildner (wie beispielsweise Fibrin), der Steroide, der Proteine, der Hormone, der Insuline, der Zytostatika, der Peptide, der Vasodilatatoren (wie beispielsweise Sartane) und der antiproliferativ wirkenden Stoffe, beispielsweise Paclitaxel oder Sirolimus, bestehen.

Derzeit werden auch intraluminale Endoprothesen eingesetzt, die aus einem Material bestehen, das der Biodegradation unterliegt. Hierbei werden unter Biodegradation hydrolytische, enzymatische oder andere stoffwechselbedingte Abbauprozesse im lebenden Organismus verstanden, die vor allem durch die mit der Endoprothese in Kontakt gelangenden Körperflüssigkeiten verursacht werden und zu einer allmählichen Auflösung zumindest großer Teile der Endoprothese führen. Synonym zu dem Begriff Biodegradation wird häufig der Begriff Biokorrosion verwendet. Der Begriff Bioresorption umfasst die anschließende Resorption der Abbauprodukte durch den lebenden Organismus. Derartige biodegradierbare Materialien können aus Polymeren oder Metallen realisiert werden. Im Zusammenhang mit Stents ist auch die Abkürzung AMS (Absorbierbarer Metall Stent) gebräuchlich. Derartige Stents enthalten ein biodegradierbares Metall, vorzugsweise Magnesium und/oder eine Magnesiumlegierung.

Es ist außerdem bekannt, intraluminale Endoprothesen mit Funktionselementen zu versehen, die zumindest in einem Teil ihres Volumens eine verglichen mit dem Material des Grundgitters verschiedene Materialzusammensetzung aufweisen. Diese Funktionselemente dienen beispielsweise der Bestimmung der Position einer Endoprothese im Körper oder dem Freisetzen von Medikamenten.

Die Ermittlung der Position einer Endoprothese geschieht häufig mittels bildgebender Verfahren, beispielsweise mittels einer Röntgenstrahleinrichtung. Da die für das Grundgitter von derartigen Endoprothesen verwendeten Materialien in der Regel Röntgenstrahlung nur in geringem Maße absorbieren, d. h. röntgen- oder radioluzent sind, werden die Endoprothesen häufig mit sogenannten Röntgenmarkern versehen, die ein Material enthalten, das eine höhere Absorption der Röntgenstrahlen aufweist (röntgenopakes oder radioopakes Material).

Bei einem Katheter, der zum Einbringen einer intraluminalen Endoprothese, beispielsweise eines Stents, dient, besteht häufig das Problem, das an den Enden eines auf einem solchen Katheter aufgebrachten Stents Steifigkeitssprünge entstehen. Insbesondere wenn der Stent steif ist oder durch einen großen Crimpdurchmesser eine hohe Steifigkeit aufweist, besteht die Gefahr, dass der Katheter in Kurven des Körperhohlraums, den er passiert, an den Steifigkeitssprüngen abgeknickt wird und so den Führungsdraht einklemmt. Dadurch kann die Reibung zwischen Katheter und Führungsdraht ansteigen. Weist der Stent zusätzlich Röntgenmarker an den Stentenden auf, besteht die Gefahr, dass diese in Kurven durch das Abknicken des Katheters abstehen und so im Körperhohlraum oder am Führungskatheter hängen bleiben.

Aus der Druckschrift US 7,022,106 B2 ist ein Ballonkatheter bekannt, bei dem eine Erhöhung der Vorschubkraft, die von dem Außenschlauch auf das distale Ende des Katheters übertragen wird, erreicht wird. Hierfür weist der bekannte Katheter einen Außenschlauch und einen Innenschlauch auf, der sich koaxial in dem Außenschlauch erstreckt. Außerdem ist ein Ballon in der Nähe des distalen Endes des Katheters angeordnet. Der Ballon ist an seinem proximalen Befestigungspunkt an dem Außenschlauch befestigt und an seinem distalen Befestigungspunkt mit dem Innenschlauch verbunden. Außerdem kann der Außenschlauch, um seine Steifigkeit und Pushability entlang dieses Segments des Katheter zu erhöhen, verstärkt sein. In einer Ausführungsform des bekannten Katheters verjüngt sich der Außenschlauch schrittweise ausgehend von dem proximalen Befestigungspunkt in distale Richtung und bildet einen konusförmigen Abschnitt. Der Nachteil dieses bekannten Katheters besteht darin, dass der Aufbau recht kompliziert gestaltet ist, so dass der Katheter in der Herstellung kostenintensiv ist. Zudem löst der bekannte Katheter nicht das oben geschilderte Problem, da die Steifigkeitssprünge an den Stentenden durch die bekannte Konstruktion nicht beseitigt werden.

Ein weiterer bekannter Katheter ist in der Druckschrift US 2007/0016132 A1 beschrieben. Dieser Katheter weist einen länglichen Körper mit einem proximalen Abschnitt und einem distalen Abschnitt auf. Weiterhin sind an dem Katheter eine Vielzahl von Versteifungsmitteln vorgesehen, welche zur Variation der Steifigkeit entlang des Katheterkörpers dienen. In der Druckschrift sind verschiedene Typen von Versteifungsmitteln erläutert, die beispielsweise überlappen oder entlang der Länge des Katheterkörpers eine unterschiedliche Steifigkeit aufweisen. Hierfür können die entlang der Länge des Körpers des Katheters angeordneten Versteifungsmittel aus unterschiedlichen Materialen bestehen, die eine unterschiedliche Flexibilität aufweisen. Die Druckschrift beschäftigt sich allerdings nicht mit dem oben geschilderten Problem der Steifigkeitssprünge an den Endoprothesenenden, wenn mittels des Katheters eine intraluminale Endoprothese in einen Körperhohlraum eingebracht wird.

EP1327422 offenbart einen Ballonkatheter zur Einführung einer intraluminalen Endoprothese. Im Bereich mindestens eines Ende des zur Anordnung der Endoprothese geeigneten Ballonabschnitts befindet sich ein Versteifungsmittel, wodurch ein Steifigkeitsübergang zwischen Endoprothese und Katheter erreicht werden kann.

Aus US6702802 B1 geht ein weiterer Ballonkatheter hervor, wobei konische, teilweise aus röntgenopakem Material bestehende Schlauchelemente die Steifigkeitssprünge an den Endbereichen einer auf dem Ballon angeordneten Endoprothese beseitigen.

Die Aufgabe der vorliegenden Erfindung besteht demnach darin, einen Katheter zum Einbringen einer intraluminalen Endoprothese zu schaffen, bei dem Steifigkeitssprünge vermieden werden. Die Aufgabe besteht ferner darin, ein entsprechendes System zum Einbringen einer intraluminalen Endoprothese zu schaffen.

Die obige Aufgabe wird durch einen Katheter gelöst, bei dem im Bereich mindestens eines Endes des Endoprothesenabschnitts in dessen Längsrichtung auf dem Innenschlauch ein Versteifungsmittel angeordnet ist. Als Längsrichtung wird hierbei die Richtung be-zeichnet, in der sich die Längsachse des Katheters erstreckt. Der angegebene Bereich des Endes des Endoprothesenabschnitts umfasst dabei sowohl einen kurzen Abschnitt mit einer Länge von etwa 2 mm bis etwa 15 mm des Katheters, der in Längsrichtung an den Endoprothesenabschnitt angrenzt, aber außerhalb des Endoprothesenabschnitts liegt, als auch den jeweils angrenzenden Endabschnitt des Endoprothesenabschnitts. Durch ein derartiges Versteifungsmittel, das im Bereich eines Endes oder beider Enden des Endoprothesenabschnitts in dessen Längsrichtung angeordnet ist, wird der Innenschlauch des Katheters lokal versteift und ein im Wesentlichen kontinuierlicher Steifigkeitsübergang des gesamten Bereichs bestehend aus Innenschlauch, Ballon und Stent geschaffen, so dass in diesem Bereich oder diesen Bereichen kein Steifigkeitssprung mehr vorliegt.

In einem aus dem Stand der Technik bekannten Beispiel ist das Versteifungsmittel als Polymerschlauch ausgebildet, der vorzugsweise mit dem Innenschlauch verschweißt ist oder auf den Innenschlauch aufgeklebt ist. Ein derartiges Versteifungsmittel kann besonders kostengünstig hergestellt werden sowie einfach und positionsgenau auf dem Innenschlauch angeordnet werden.

Vorzugsweise enthält der Polymerschlauch ein oder mehrere Polymere aus der Gruppe Polyamide und Pebax. Diese Polymere haben besonders gute Eigenschaften in Bezug auf das Zusammenwirken mit den übrigen Komponenten des Katheters.

In einem weiteren bekannten Beispiel weist der Polymerschlauch einen Außendurchmesser auf, der sich von seinem Ende in Längsrichtung, das außerhalb des Endoprothesenabschnitts angeordnet ist, bis zu seinem Ende in Längsrichtung, das innerhalb oder genau an der Kante des Endoprothesenabschnitts angeordnet ist, im Wesentlichen kontinuierlich vergrößert. Die Vergrößerung des Außendurchmessers ist verbunden mit einer der Vergrößerung des Außendurchmessers entsprechenden Vergrößerung der Wandstärke des Polymerschlauchs, so dass in diese Richtung ebenfalls die Steifigkeit des Polymerschlauchs zunimmt. Hierdurch kann ein besonders einfacher und kostengünstiger Steifigkeitsübergang in den Bereichen der Enden des Endoprothesenabschnitts realisiert werden.

Ebenfalls bevorzugt ist es, wenn das Material des Polymerschlauchs mindestens ein röntgenopakes (röntgendichtes) Material enthält, beispielsweise einen Füllstoff aus Barium, Bismut oder Wolfram. Dieses Ausführungsbeispiel ermöglicht es, auf einen separaten Röntgenmarker, der z. B. aus Platin besteht, zu verzichten.

In einem Ausführungsbeispiel der Erfindung weist das Versteifungsmittel mehrere Metallringe auf, die in einer vorbestimmten, an den durch die jeweilige intraluminale Endoprothese erzeugten Steifigkeitssprung angepassten Länge und/oder Abstand vorgesehen sind, wobei der eine Metallring oder die Metallringe vorzugsweise auf den Innenschlauch aufgecrimpt sind. Durch eine solche Anordnung, insbesondere von mehreren Metallringen, im Bereich des Endes des Endoprothesenabschnitts lässt sich ebenfalls durch einfache, kostengünstige Mittel ein Steifigkeitsübergang erzielen, wobei vorzugsweise Materialien verwendet werden, die bei Röntgenbestrahlung sichtbar sind, so dass diese Ringe als Markierungsringe zur Röntgensichtbarkeit verwendet werden können.

Besonders bevorzugt enthalten die Metallringe eine oder mehrere Metalle aus der Gruppe Platin und Iridium. Diese Metalle weisen besonders gute Steifigkeitseigenschaften auf und können unter Röntgenbestrahlung als Markierung dienen.

Besonders bevorzugt sind bis 5 Metallringe vorgesehen, die eine Länge von etwa 0,1 mm bis 1 mm und/oder einen Abstand von etwa 0,1 mm bis 1 mm aufweisen.

Die obige Aufgabe wird außerdem durch ein System zum Einbringen einer intraluminalen Endoprothese gelöst, das aus der intraluminalen Endoprothese und einem Katheter besteht, wobei der Katheter oben beschriebene Eigenschaften aufweist und wobei die intraluminale Endoprothese in dem Endoprothesenabschnitt auf dem Ballon angeordnet ist, wobei die Endoprothese vorzugsweise außen auf dem Endoprothesenabschnitt des Ballons aufgecrimpt ist. Mit einem derartigen System werden Steifigkeitssprünge an den Enden der Endoprothese vermieden und ein Steifigkeitsübergang geschaffen, der ein Abknicken des Katheters beim Einbringen der Endoprothese in den Körperhohlraum vermeidet. Ebenfalls kann durch ein derartiges erfindungsgemäßes System ein Hängenbleiben von an den Enden der Endoprothese angeordneten Röntgenmarkern im Körperhohlraum vermieden werden.

In einem bevorzugten Ausführungsbeispiel weist die Endoprothese mindestens an einem an ihren in Längsrichtung angeordneten Enden einen Röntgenmarker auf. Durch einen derartigen Röntgenmarker kann bei einer Bestrahlung mittels Röntgenstrahlung der Ort der Endoprothese nachgewiesen und kontrolliert werden.

In einem weiteren bevorzugten Ausführungsbeispiel weist die Endoprothese eine pharmazeutisch aktive Substanz auf, wodurch in vorteilhafter Weise die pharmazeutisch aktive Substanz direkt am Ort der Einsetzung der Endoprothese eingebracht werden kann und hierdurch Nebenwirkungen vermieden werden. Alternativ oder zusätzlich kann die Endoprothese mindestens teilweise aus einem degradierbaren Material bestehen.

Weitere Ziele, Merkmale, Vorteile und Anwendungsmöglichkeiten der Erfindung ergeben sich aus der nachfolgenden Beschreibung von Ausführungsbeispielen anhand der Figuren. Dabei bilden alle beschriebenen und/oder bildlich dargestellten Merkmale für sich oder in beliebiger Kombination den Gegenstand der vorliegenden Erfindung, auch unabhängig von ihrer Zusammenfassung in den einzelnen Ansprüchen oder deren Rückbeziehung.

Es zeigen schematisch:
- Fig. 1a: ein erstes Beispiel eines Systems bestehend aus einem Katheter, der im Längsschnitt dargestellt ist und einem Stent, der in einer Ansicht von der Seite gezeigt ist,
- Fig. 1b: einen Längsschnitt eines Versteifungsmittels des in Fig. 1a dargestellten Aus- führungsbeispiels eines Systems und
- Fig. 2: ein Ausführungsbeispiel eines erfindungsgemäßen Systems aus erfin- dungsgemäßem Katheter und Stent, wobei der Katheter in einem Längsschnitt und der Stent in einer Ansicht von der Seite gezeigt ist.

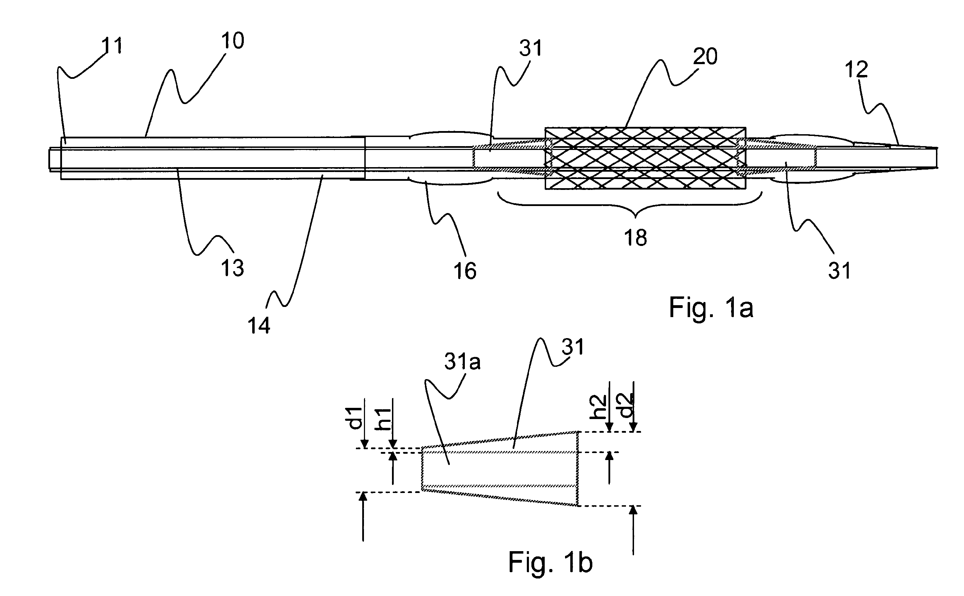

In Fig. 1a ist ein distaler Endabschnitt eines Katheters 10 mit einem proximalen Ende 11 und einem distalen Ende 12 des Endabschnitts im Längsschnitt gezeigt. Der Katheter 10 weist einen nicht dargestellten Führungsdraht sowie einen Innenschlauch 13 und einen Außenschlauch 14 auf. Der Innenschlauch 13 ist koaxial innerhalb des Außenschlauchs 14 angeordnet, so dass der Außenschlauch 14 den Innenschlauch 13 umgibt.

An den Außenschlauch 14 ist an seinem distalen Ende ein Ballon 16 angeschweißt oder auf andere Weise mit diesem verbunden bzw. an diesem befestigt, wobei der Ballon bei einer Befüllung mit einem Kontrastmittel/Kochsalzlösungsgemisch im Durchmesser erweitert werden kann. Der Ballon 16 umgibt ebenfalls den Innenschlauch 13. An dem Ballon 16 ist der Endoprothesenabschnitt 18 vorgesehen, in dem der Stent 20 angeordnet ist. Der Stent 20 ist auf den Ballon 16 im Bereich des Endoprothesenabschnitts 18 des Katheters 10 aufgecrimpt.

An dem in Längsrichtung angeordneten distalen und proximalen Ende des Endoprothesenabschnitts 18 weist der Innenschlauch 13 je einen mit dem Innenschlauch 13 verschweißten Polymerschlauch 31 als Versteifungsmittel auf, der im Längsschnitt in Fig. 1b näher dargestellt ist.

Der Polymerschlauch 31 ist in seiner äußeren Form im Wesentlichen kegelstumpfförmig ausgebildet und weist an einem Ende einen ersten Außendurchmesser d1 und an einem zweiten Ende einen zweiten Außendurchmesser d2 auf. Hierbei ist der erste Außendurchmesser d1 kleiner als der zweite Außendurchmesser d2. Zudem hat der Polymerschlauch eine durchgehende Öffnung 31a, die zylinderförmig ausgebildet ist und die Anordnung des Polymerschlauchs 31 auf dem Innenschlauch 13 erlaubt.

Bei dem am proximalen Ende des Endoprothesenabschnitt 18 angeordneten Polymerschlauch 31 ist der erste Durchmesser d1 am proximalen Ende des Polymerschlauchs 31 angeordnet. Bei dem am distalen Ende des Endoprothesenabschnitt 18 vorgesehenen Polymerschlauch 31 befindet sich der erste Außendurchmesser d1 am distalen Ende des Polymerschlauchs 31, wobei jeweils die Enden des Polymerschlauchs 31 mit den ersten Außendurchmessern d1 geringfügig außerhalb des Endoprothesenabschnitts 18 angeordnet sind. Die Enden des Polymerschlauchs 31 mit den zweiten Außendurchmessern d2 sind dem gegenüber innerhalb des Endoprothesenabschnitts 18 angeordnet. In einem weiteren Ausführungsbeispiel können die Enden des Polymerschlauchs 31 mit den zweiten Außendurchmessern d2 auch direkt an der Kante des Endoprothesenabschnitts 18 angeordnet sein. Im Bereich des ersten Außendurchmessers d1 weist der Polymerschlauch 31 eine erste Wandstärke h1 und im Bereich des zweiten Außendurchmessers d2 eine zweite Wandstärke h2 auf. Die Wandstärke h1 ist kleiner als die zweite Wandstärke h2, so dass im Bereich des ersten Außendurchmessers d1 der Polymerschlauch 31 eine geringere Steifigkeit als im Bereich des zweiten Außendurchmessers d2 aufweist. Die Steifigkeit nimmt entlang der Längsrichtung des Polymerschlauchs 31, d.h. entlang der Richtung seiner Längsachse, von dem Ende des Polymerschlauchs 31 mit dem ersten Außendurchmesser d1 bis zu dem Bereich mit dem zweiten Außendurchmesser d2 kontinuierlich zu.

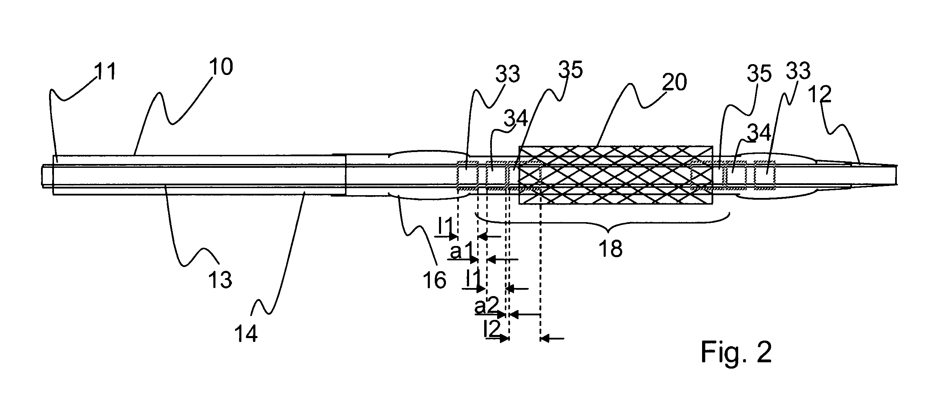

In dem in Fig. 2 dargestellten Ausführungsbeispiel des erfindungsgemäßen Systems aus Katheter 10 und Stent 20 sind statt der Polymerschläuche 31 als Versteifungsmittel jeweils in Längsrichtung an beiden Enden des Endoprothesenabschnitts 18 drei Metallringe 33, 34 und 35 vorgesehen. Die Metallringe 33, 34 und 35 enthalten vorzugsweise mindestens ein Metall der Gruppe Platin und Iridium und sind vorzugsweise auf den Innenschlauch 13 aufgecrimpt.

Die Metallringe 33, 34 und 35 sind am proximalen Ende des Endoprothesenabschnitts 18 in dieser Reihenfolge in Richtung des distalen Endes angeordnet. Am distalen Ende des Endoprothesenabschnitts 18 sind die Metallringe 33, 34 und 35 in umgekehrter Reihenfolge angeordnet, wobei die Metallringe 33 und 34 an beiden Enden jeweils teilweise außerhalb des Endoprothesenabschnitts 18 angeordnet sind. Der Metallring 35 ist jeweils innerhalb des Endoprothesenabschnitts 18 vorgesehen. Die Metallringe 33 und 34 weisen jeweils eine Länge 11 in Längsrichtung von 0,5 mm, während der Metallring 35 eine Länge 12 von 1 mm aufweist. Die Metallringe 33 und 34 haben einen Abstand a1 von 0,5 mm und die Metallringe 34 und 35 einen Abstand a2 von 0,3 mm. Durch eine derartige Wahl der Länge der Metallringe 33, 34 und 35 bzw. des Abstandes dieser Metallringe kann ein besonders vorteilhafter Steifigkeitsübergang an den Enden des Endoprothesenabschnitts 18 erreicht werden.

### Bezugszeichenliste:

- 10: Katheter
- 11: proximales Ende des distalen Endabschnitts des Katheters 10
- 12: distales Ende des distalen Endabschnitts des Katheters 10
- 13: Innenschlauch
- 14: Außenschlauch
- 16: Ballon
- 18: Endoprothesenabschnitt des Ballons 16
- 20: Stent
- 31: Polymerschlauch
- 31a: durchgehende Öffnung des Polymerschlauchs 31
- 33, 34, 35: Metallring
- d1: erster Außendurchmesser des Polymerschlauchs 31
- d2: zweiter Außendurchmesser des Polymerschlauchs 31
- h1: erste Wandstärke des Polymerschlauchs 31
- h2: zweite Wandstärke des Polymerschlauchs 31
- 11: Länge der Metallringe 33, 34
- 12: Länge des Metallrings 35
- a1: Abstand zwischen den Metallringen 33 und 34
- a2: Abstand zwischen den Metallringen 34 und 35

## Patentansprüche

1. Katheter (10) mit einem Innenschlauch (13) und einem den Innenschlauch (13) zumindest abschnittsweise umgebenden Außenschlauch (14), wobei der Außenschlauch (14) mit einem Ballon (16) verbunden ist, der einen Endoprothesenabschnitt (18) aufweist, welcher zur Anordnung einer intraluminalen Endoprothese (20) vorgesehen ist, und wobei im Bereich mindestens eines Endes des Endoprothesenabschnitts (18) in dessen Längsrichtung auf dem Innenschlauch (13) ein Versteifungsmittel (31, 33, 34, 35) angeordnet ist, **dadurch gekennzeichnet, dass** das Versteifungsmittel mehrere Metallringe (33, 34, 35) aufweist, die in einer vorbestimmten, an den durch die jeweilige intraluminale Endoprothese (20) erzeugten Steifigkeitssprung angepassten Länge (11, 12) und/oder Abstand (a1, a2) vorgesehen sind, wobei die Metallringe (33, 34, 35) vorzugsweise auf den Innenschlauch (13) aufgecrimpt sind.

2. Katheter (10) nach Anspruch 1, **dadurch gekennzeichnet, dass** das Versteifungsmittel (31, 33, 34, 35) in den Bereichen beider Enden des Endoprothesenabschnitts (18) in dessen Längsrichtung angeordnet ist.

3. Katheter (10) nach Anspruch 1 oder 2, **dadurch gekennzeichnet, dass** die Metallringe (33, 34, 35) ein oder mehrere Metalle aus der Gruppe Platin und Iridium enthalten.

4. Katheter (10) nach einem der Ansprüche 1 bis 3, **dadurch gekennzeichnet, dass** als Versteifungsmittel 2 bis 5 Metallringe (33, 34, 35) vorgesehen sind, die eine Länge (11, 12) von etwa 0,1 mm bis 1 mm und/oder einen Abstand (a1, a2) von etwa 0,1 mm bis 1 mm aufweisen.

5. System zum Einbringen einer intraluminalen Endoprothese (20), vorzugsweise eines Stents, in einen Körperhohlraum bestehend aus der intraluminalen Endoprothese (20) und einem Katheter (10) nach einem der vorhergehenden Ansprüche, wobei die intraluminale Endoprothese (20) in dem Endoprothesenabschnitt (18) auf dem Ballon (16) angeordnet ist, wobei die Endoprothese (20) vorzugsweise außen auf dem Endoprothesenabschnitt (18) des Ballons (16) aufgecrimpt ist.

6. System nach Anspruch 5, **dadurch gekennzeichnet, dass** die Endoprothese (20) mindestens an einem an ihren in Längsrichtung angeordneten Enden ein Röntgenmarker aufweist.

7. System nach einem der Ansprüche 5 oder 6, **dadurch gekennzeichnet, dass** die Endoprothese (20) eine pharmazeutisch aktive Substanz aufweist.

## Claims

1. A catheter (10) comprising an inner tube (13) and an outer tube (14) which encloses the inner tube (13) at least in sections, wherein the outer tube (14) is connected to a balloon (16) comprising an endoprothesis section (18) used to position an intraluminal endoprothesis (20), and wherein a stiffening member (31, 33, 34, 35) is disposed on the inner tube (13) in the region of at least one end of the endoprothesis section (18) in the longitudinal direction thereof, **characterized in that** the stiffening member comprises a plurality of metal rings (33, 34, 35) which are provided in a predetermined length (11, 12) and/or separation (a1, a2) adapted to the abrupt change in stiffness resulting from the particular intraluminal endoprosthesis (20), wherein the metal rings (33, 34, 35) are preferably crimped onto the inner tube (13).

2. The catheter (10) according to claim 1, **characterized in that** the stiffening member (31, 33, 34, 35) is disposed in the regions of both ends of the endoprothesis section (18) in the longitudinal direction thereof.

3. The catheter (10) according to claim 1 or 2, **characterized in that** the metal rings (33, 34, 35) contain one or more metals from the group comprising platinum and iridium.

4. The catheter (10) according to one of the claims 1 to 3, **characterized in that**, as stiffening members, 2 to 5 metal rings (33, 34, 35) are provided, which have a length (11, 12) of approximately 0.1 mm to 1 mm and/or a separation (a1, a2) of approximately 0.1 mm to 1 mm.

5. A system for delivering an intraluminal endoprosthesis (20), preferably a stent, into a body cavity, comprising the intraluminal endoprosthesis (20) and a catheter (10) according to one of the preceding claims, wherein the intraluminal endoprosthesis (20) is disposed on the balloon (16) in the endoprosthesis section (18), wherein the endoprosthesis (20) is preferably crimped onto the outside of the endoprosthesis section (18) of the balloon (16).

6. The system according to claim 5, **characterized in that** the endoprosthesis (20) comprises an x-ray marker on at least one end thereof disposed in the longitudinal direction.

7. The system according to one of the claims 5 or 6, **characterized in that** the endoprosthesis (20) comprises a pharmaceutically active substance.

## Revendications

1. Cathéter (10) avec un tube interne (13) et un tube externe (14) entourant au moins par tronçons le tube interne (14), dans lequel le tube externe (14) est relié à un ballon (16) comportant une section d'endoprothèse (18) prévue pour la disposition d'une endoprothèse intraluminale (20), et dans lequel un moyen de rigidification (31, 33, 34, 35) est disposé sur le tube interne (13) dans la région d'au moins une extrémité de la section d'endoprothèse (18), dans le sens longitudinal de celle-ci, **caractérisé en ce que** le moyen de rigidification comporte plusieurs anneaux métalliques (33, 34, 35) prévus dans une longueur (11, 12) et/ou un espacement (a1, a2) prédéfinis adaptés à l'écart de rigidité produit par chacune des prothèses intraluminales (20), les anneaux métalliques (33, 34, 35) étant de préférence sertis sur le tube interne (13).

2. Cathéter (10) selon la revendication 1, **caractérisé en ce que** le moyen de rigidification (31, 33, 34, 35) est disposé dans les régions des deux extrémités de la section d'endoprothèse (18), dans le sens longitudinal de celle-ci.

3. Cathéter (10) selon la revendication 1 ou 2, **caractérisé en ce que** les anneaux métalliques (33, 34, 35) contiennent un ou plusieurs métaux issus du groupe platine et iridium.

4. Cathéter (10) selon l'une des revendications 1 à 3, **caractérisé en ce que** 2 à 5 anneaux métalliques (33, 34, 35), d'une longueur (11, 12) d'environ 0,1 mm à 1 mm et/ou avec un espacement (a1, a2) d'environ 0,1 mm à 1 mm, sont prévus en tant que moyens de rigidification.

5. Système pour l'introduction d'une endoprothèse intraluminale (20), de préférence d'un implant, dans un espace creux du corps, constitué d'une endoprothèse intraluminale (20) et d'un cathéter (10) selon l'une des revendications précédentes, dans lequel l'endoprothèse intraluminale (20) est disposée sur un ballon (16) dans une section d'endoprothèse (18), l'endoprothèse (20) étant de préférence sertie à l'extérieur sur la section d'endoprothèse (18) du ballon (16).

6. Système selon la revendication 5, **caractérisé en ce que** l'endoprothèse (20) comporte un marqueur radio-opaque à l'une de ses extrémités disposées dans le sens longitudinal.

7. Système selon l'une des revendications 5 ou 6, **caractérisé en ce que** l'endoprothèse (20) comporte une substance pharmaceutique active.
